# FASCICULE DE BREVET EUROPEEN

(11) **EP 0 526 306 B1**
(45) Date de publication et mention de la délivrance du brevet: **08.01.1997**
(21) Numéro de dépôt: 92402107.4
(22) Date de dépôt: 21.07.1992
(51) Int. Cl.: H05H 9/02

(54) **Accélérateur de protons à l'aide d'une onde progressive à couplage magnétique**
Wanderwellen-Protonbeschleuniger mit magnetischer Kupplung
Magnetically coupled travelling wave proton accelerator

(30) Priorité: 23.07.1991 FR 9109292
(43) Date de publication de la demande: 03.02.1993
(73) Titulaire: CGR MEV, F-78530 Buc (FR)
(72) Inventeur: Tronc, Dominique, F-75116 Paris (FR)
(74) Mandataire: Ballot, Paul Denis Jacques

(56) Documents cités:
- DE-A- 3 610 584
- FR-A- 2 127 228
- FR-A- 2 576 477
- FR-A- 2 656 192
- US-A- 2 842 705
- US-A- 3 068 425
- US-A- 3 953 758
- IEEE TRANSACTIONS ON NUCLEAR SCIENCE. vol. NS-32, no. 5, Octobre 1985, NEW YORK US pages 3243 - 3245 TRONC ET AL. 'Electron LINAC optimisation for short RF and beam pulse lengths'
- Nuclear Instr. and Methods in Physics Research, A327 (1993) 253- 255, North-Holland

## Description

L'invention concerne les accélérateurs de protons en vue d'obtenir un faisceau de grande énergie.

Afin de soigner les tumeurs malignes, il est connu de les soumettre à un rayonnement plus ou moins intense et on a donc recours à des sources de rayons X ou de particules chargées tels que des électrons ou des protons. Dans le cas des particules chargées, les protons présentent l'avantage d'une meilleure définition du volume-cible par suite, d'une part, de leur pénétration à profondeur bien définie (pic de Bragg) et, d'autre part, de l'absence de pénombre.

Aussi, il est de plus en plus proposé d'utiliser des accélérateurs de protons mais l'énergie requise est très élevée compte tenu de leur pénétration-réduite dans la matière, de l'ordre de 10 centimètres par 100 MeV d'énergie. Aussi, pour réaliser un accélérateur linéaire de protons de 250 MeV du type classique à onde stationnaire, on est conduit à des longueurs qui peuvent atteindre vingt-huit mètres. Un tel accélérateur de protons a, par exemple, été décrit lors de la présentation à la conférence "Particle Accelerator Conference" par R.W. HAMM, K.R. CRANDALL, et J.M. POTTER qui s'est tenue du 6 au 9 mai 1991 à SAN FRANCISCO, ladite présentation étant intitulée : PRELIMINARY DESIGN OF A DEDICATED PROTON THERAPY LINAC.

Un but de la présente invention est donc de réaliser un accélérateur de protons à usage médical ou autre dont les dimensions sont réduites dans un facteur deux à trois et le coût dans un facteur encore plus important du fait de la simplicité de la structure mise en oeuvre. Il est à noter que des applications autres que médicales peuvent tirer avantage de la simplification apportée par la présente invention au prix d'une adaptation particulière, notamment en ce qui concerne le diamètre de passage du faisceau de protons qui sera porté à plus de dix millimètres au lieu des quatre millimètres pour une application médicale.

Pour atteindre ce but, l'invention met en oeuvre une ou plusieurs structures accélératrices du type à onde progressive à couplage magnétique, soit à propagation directe de l'onde accélératrice, soit à propagation inverse de ladite onde, par rapport au sens de propagation du faisceau de protons.

Un accélérateur linéaire de particules chargées, notamment d'électrons, à onde progressive à couplage magnétique et à propagation inverse est connu et est, par exemple, décrit dans le brevet fiançais n° 2 576 477 déposé le 18 janvier 1985 par la demanderesse ainsi que dans l'article paru dans IEEE Transactions on Nuclear Sciences, Vol-NS-32, Octobre 1985,3243 et intitulé : ELECTRON LINAC OPTIMISATION FOR SHORT RF AND BEAM PULSE LENGTHS.

Les accélérateurs linéaires de protons de type connu, notamment celui cité en premier lieu ci-dessus, comprennent une pluralité de types de lignes accélératrices telles que les cavités accélératrices linéaires dites d'ALVAREZ et les lignes à onde stationnaire. Ces dernières sont limitées en longueur, ce qui conduit à leur multiplicité, par exemple dix dans l'exemple de réalisation rappelé ci-dessus. Ces dernières sont souvent alimentées chacune en énergie haute fréquence par un klystron, ce qui conduit à une augmentation importante du coût de tels accélérateurs.

Un autre but de la présente invention est donc de réaliser un accélérateur linéaire de protons du type à onde progressive dans lequel un seul klystron alimente en énergie haute fréquence une ou plusieurs structures accélératrices disposées en série vu du faisceau. Les cellules de chaque structure accélératrice doivent avoir des caractéristiques adaptées à la vitesse atteinte par les protons du faisceau. Ainsi, les structures accélératrices seront du type à onde progressive à propagation directe ou inverse et leur mode sera dit fondamental ou harmonique.

L'invention concerne un accélérateur linéaire de protons du type à onde progressive pour obtenir un faisceau de protons d'une énergie déterminée qui comporte une source fournissant un faisceau de protons, se propageant à une vitesse inférieure à la lumière suivant une direction donnée, au moins une structure accélératrice du type à onde progressive à couplage magnétique disposée de manière que le faisceau de protons issu de ladite source pénètre dans ladite structure accélératrice et en sorte à l'énergie déterminée, et au moins un klystron fournissant l'énergie haute fréquence à ladite structure accélératrice, caractérisé en ce que :
- ladite structure accélératrice est constituée de cellules dont la longueur est variable suivant la direction de propagation dudit faisceau de protons afin de tenir compte de la variation de la vitesse des protons au fur et à mesure de leur accélération dans ladite structure.
- Ladite structure accélératrice est du type à propagation directe en mode harmonique présentant un déphasage préférentiel de (π/2-2π) ou (3π/4-2π) ou à propagation inverse en mode fondamental de déphasage préférentiel égal à 3π/4 ou en mode harmonique de déphasage préférentiel égal à (π/2+2π).

Selon l'invention, plusieurs structures accélératrices de types différents peuvent être disposées en série par rapport au faisceau de protons de manière à obtenir un faisceau de protons ayant l'énergie souhaitée.

D'autres buts, caractéristiques et avantages de la présente invention apparaîtront à la lecture de la description suivante d'un exemple particulier de réalisation, ladite description étant faite en relation avec les dessins joints dans lesquels :
- la figure 1 est une vue schématique d'un premier mode de réalisation d'un accélérateur linéaire de protons du type à onde progressive à couplage magnétique selon l'invention;
- la figure 2 est une vue en coupe schématique d'un premier mode de réalisation de cellules de la structure accélératrice 12 de la figure 1 du type à onde progressive inverse à couplage magnétique;
- la figure 3 est une vue en coupe schématique d'un mode de réalisation de cellules de la structure accélératrice 13 de la figure 1 du type à onde progressive directe à couplage magnétique;
- la figure 4 est une vue en coupe schématique d'un mode de réalisation de cellules de la structure accélératrice 14 de la figure 1 du type à onde progressive inverse à couplage magnétique;
- la figure 5 est une vue en coupe schématique d'un deuxième mode de réalisation de cellules de la structure accélératrice 12 de la figure 1 du type à onde progressive inverse à couplage magnétique;
- la figure 6 est une vue en coupe schématique d'un mode de réalisation de deux becs successifs de la structure accélératrice 12 dont la forme particulière a pour but de focaliser le faisceau de protons;
- la figure 7 est un diagramme de BRILLOUIN en couplage magnétique montrant les divers modes qui peuvent être utilisés pour l'accélération par une onde progressive à couplage magnétique à propagation directe ou inverse et suivant un mode dit fondamental ou harmonique;
- la figure 8-a est une vue en coupe schématique d'une structure accélératrice qui comporte des espaces de glissement de manière à allonger les cellules d'accélération selon l'axe;

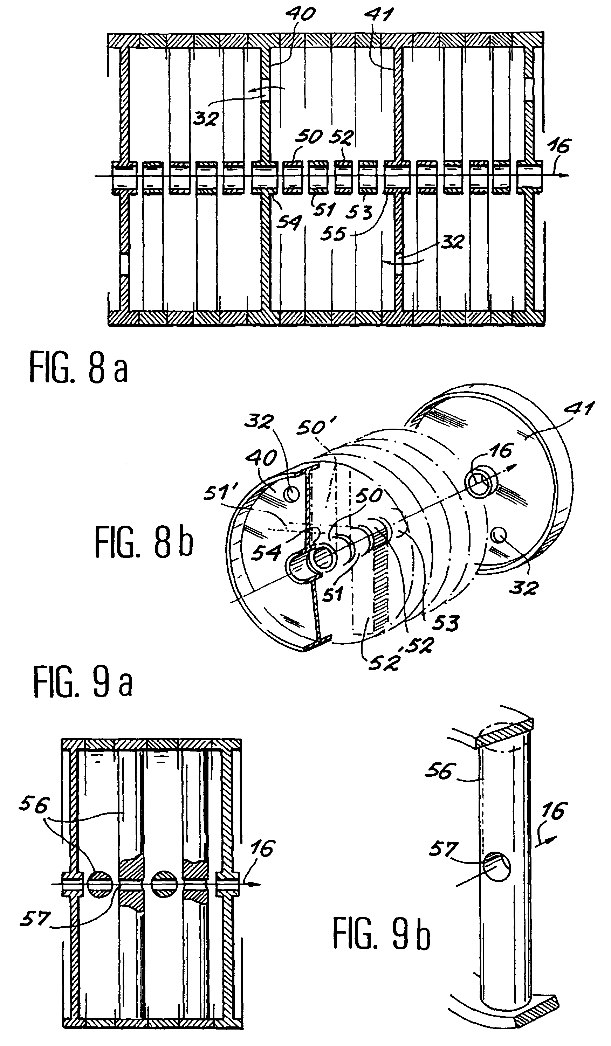

- la figure 8-b est une vue en perspective d'une cellule selon le mode de réalisation de la figure 8-a;
- la figure 9-a est une vue en coupe schématique d'un autre mode de réalisation des espaces de glissement, pour obtenir une cellule agrandie;
- la figure 9-b est une vue en perspective d'une tige forée qui est utilisée pour réaliser un espace de glissement,

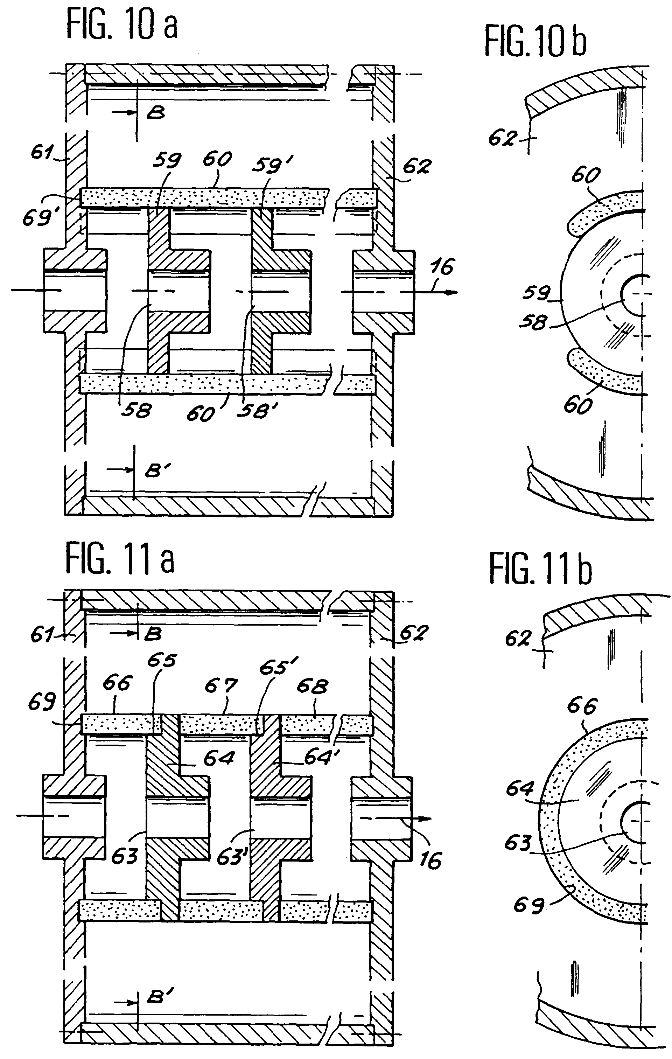

- les figures 10-a, 10-b et 11-a, 11b sont des vues en coupe montrant des formes particulières pour réaliser des espaces de glissement, et

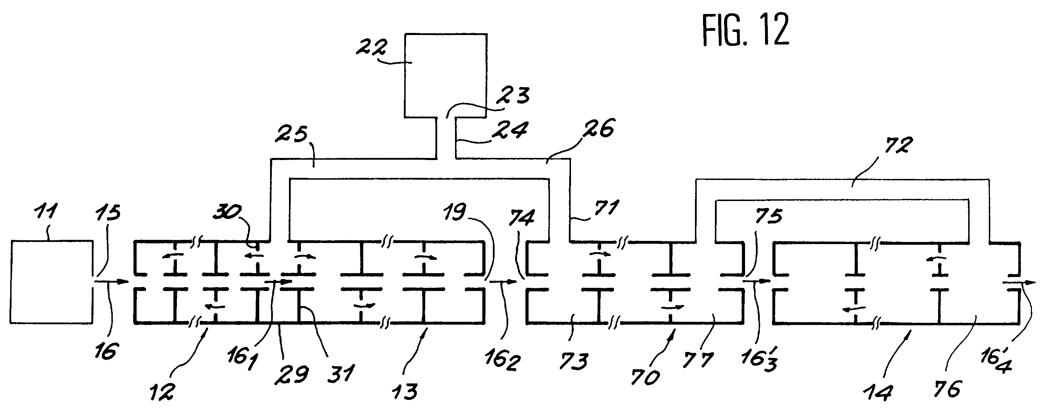

- la figure 12 est une vue schématique d'un deuxième mode de réalisation d'un accélérateur linéaire de protons selon l'invention.

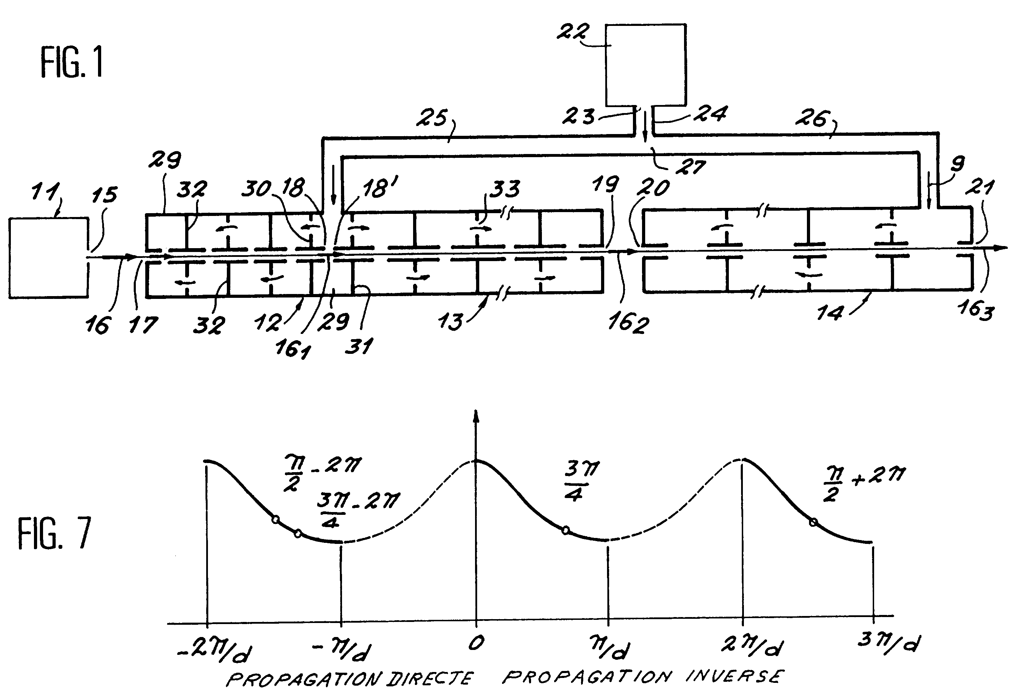

Sur la figure 1, une source de protons 11 de type classique, fournit sur sa sortie 15 un faisceau de protons, matérialisé par la flèche 16, flèche qui indique également le sens de propagation dudit faisceau. Le faisceau de protons 16, qui est fourni par la source de protons 11 et a par exemple une énergie voisine du MeV, pénètre dans une première structure accélératrice 12 par un orifice d'entrée 17 où il est soumis à une première pluralité d'accélérations de manière à atteindre une certaine énergie, par exemple 10 MeV, à un orifice de sortie 18 (flèche 16₁). Le faisceau de sortie 16₁ pénètre dans une seconde structure accélératrice 13 par un orifice d'entrée 18 qui soumet le faisceau de protons 16 à une seconde pluralité accélérations de manière à atteindre à un orifice de sortie 19 une énergie supérieure à l'énergie d'entrée, par exemple 100 MeV (flèche 16₂). Enfin, le faisceau de protons 16₂, issu de l'orifice de sortie 19, pénètre dans une troisième structure accélératrice 14 par un orifice d'entrée 20 où il est soumis à une troisième pluralité d'accélérations de manière à atteindre une énergie supérieure à l'énergie d'entrée, par exemple 250 MeV, à un orifice de sortie 21 (flèche 16₃).

Les trois structures accélératrices 12, 13, et 14 sont toutes du type à onde progressive à couplage magnétique alors que dans les accélérateurs à protons connus, les structures accélératrices sont du type à cavités dites d'ALVAREZ et/ou à onde stationnaire.

Les trois structures accélératrices 12, 13 et 14 sont alimentées en parallèle en énergie hyperfréquence, par exemple à la fréquence 2.998 Mégahertz, par une source 22 dont la borne de sortie 23 est connectée aux différentes structures accélératrices par des guides d'ondes 24, 25 et 26. La séparation de l'énergie fournie par la source 22, entre les guides 25 et 26 est obtenue de manière classique, par exemple par un coupleur 3 décibels portant la référence 27.

Les flèches telles que celle référencée 9 indiquent le sens de propagation du signal hyperfréquence dans les guides d'onde 24, 25 et 26.

Selon l'invention, le guide d'onde 25 est connecté d'une part, à la dernière cellule de la première structure accélératrice 12 et, d'autre part, à la première cellule de la deuxième structure accélératrice 13 par l'intermédiaire d'une cellule dite de couplage magnétique 29 commune aux deux structures accélératrices 12 et 13. Cette cellule de couplage 29 est disposée entre les deux structures accélératrices 12 et 13 et induit à la fois la propagation inverse propre à la structure accélératrice 12 et la propagation directe propre à la structure accélératrice 13. Ceci résulte des couplages magnétiques réalisés sur les parois 30 et 31 de la cellule de couplage 29 qui constituent respectivement la paroi de sortie de la dernière cellule de la première structure accélératrice 12 et la paroi d'entrée de la première cellule de la deuxième structure accélératrice 13. Le couplage entre le guide d'onde 25 et la cellule de couplage 29 est du type magnétique et est réalisé à la périphérie de ladite cellule de couplage. Il est à remarquer que la géométrie de cette cellule de couplage diffère peu de celle des cellules accélératrices adjacentes.

Par ailleurs, le guide d'onde 26 est connecté à la dernière cellule de la troisième structure accélératrice 14 par un couplage magnétique réalisé à la périphérie de ladite cellule.

Le couplage entre les différentes cellules de chaque structure accélératrice est du type magnétique et est obtenu par des orifices 32 percés dans les parois séparant chaque cellule. Le sens du couplage est indiqué par des flèches 33.

La source d'énergie hyperfréquence 22 est par exemple constituée, de manière classique, d'un klystron associé à un modulateur de manière à fournir des impulsions d'une durée de 3 microsecondes environ et d'une puissance crêté de 70 mégawatts environ.

Une telle source d'énergie hyperfréquence 22 ainsi que des structures accélératrices du type à onde progressive inverse pour des particules chargées, notamment d'électrons, ont été décrites, par exemple, dans le brevet français 2 576 477 et le deuxième article précités.

Pour que les structures accélératrices 12, 13 et 14 fonctionnent en onde progressive directe ou inverse à couplage magnétique de manière à accélérer des protons ayant des vitesses différentes, il est nécessaire de prévoir, d'une part, des déphasages particuliers du signal hyperfréquence et, d'autre part, des structures accélératrices présentant des cellules qui ont des configurations différentes, notamment en ce qui concerne leurs dimensions.

Le diagramme de BRILLOUIN de la figure 7 est un moyen commode et connu de l'homme de métier pour présenter les modes d'accélération. Sur la figure 7, il est présenté dans le cas du couplage magnétique et montre les différentes valeurs possibles des déphasages selon le type de propagation, directe (à gauche) et inverse (à droite) de l'axe central Ω. Sur ce diagramme, pour le choix des déphasages, seules les parties de la courbe en trait plein sont à considérer car elles correspondent aux seuls modes d'accélération possibles.

Les valeurs des déphasages qui ont été retenues parmi d'autres valeurs possibles sont les suivantes :
- en propagation directe pour les protons ayant une vitesse moyenne : (π/2-2π) et (3π/4-2π), c'est le cas de la structure accélératrice 13 ou de la structure accélératrice 14 dans une variante (fig. 12) à celle décrite en relation avec la figure 1; la présence du terme 2π ou d'un multiple de 2π correspond au fonctionnement en mode dit harmonique.
- en propagation inverse pour les protons ayant une vitesse élevée : 3π/4, c'est le cas de la structure accélératrice 14 décrite en relation avec la figure 1; l'absence du terme 2π correspond au fonctionnement en mode dit fondamental.
- en propagation inverse pour les protons ayant une vitesse lente : (π/2+2π), c'est le cas de la structure accélératrice 12 de la figure 1.

En ce qui concerne les configurations des cellules de chaque structure accélératrice, leurs dimensions, notamment longitudinales, doivent tenir compte de la vitesse des protons et leur profil doit optimiser l'impédance shunt. Par ailleurs, on limite le diamètre du faisceau à une valeur voisine de 4 millimètres, valeur retenue pour l'accélérateur à protons cité en premier lieu dans le présent mémoire.

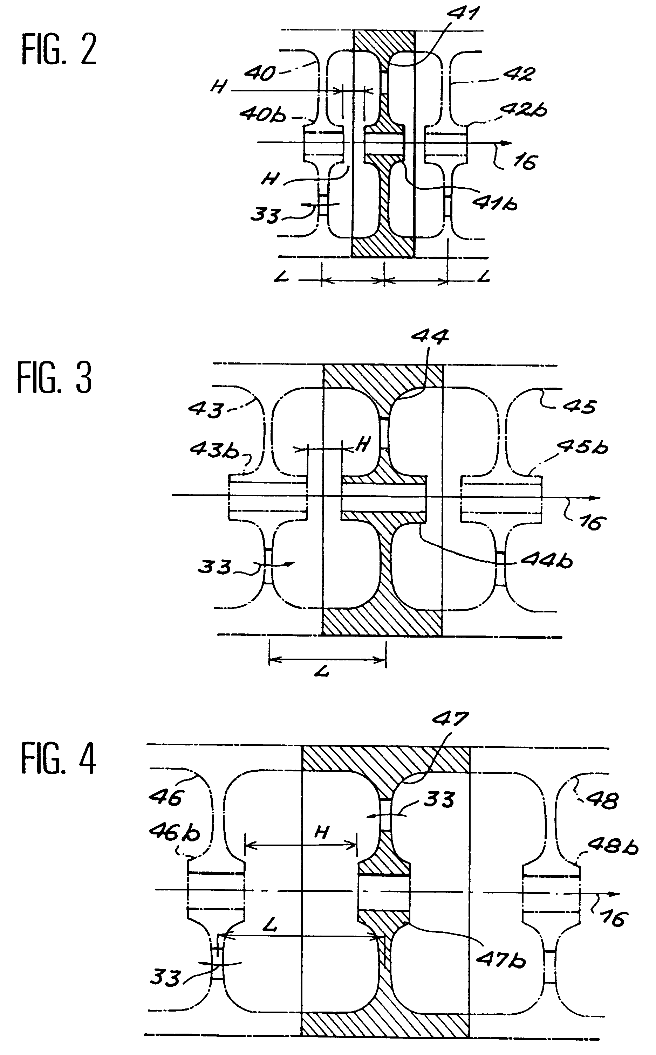

C'est ainsi que pour les protons lents (entre 0,046 et 0,103 de la vitesse de la lumière) de la première structure accélératrice 12 d'onde progressive inverse, la longueur L des cellules doit être réduite même en tenant compte du fait que les protons sont dans le tube non accélérateur pendant un intervalle de temps assez long, soit plus d'une période du signal hyperfréquence, ce qui correspond au fonctionnement en mode harmonique. La figure 2 est une vue en coupe schématique agrandie de deux cellules consécutives de la structure accélératrice 12 dans laquelle les parois transversales 40, 41 et 42 sont très rapprochées (distance L) et se terminent axialement par des becs 40b, 41b et 42b en forme de cylindre de section circulaire. La distance L et la distance inter-becs H sont données par le tableau "A" ci-après. Ce tableau fait apparaître que les cellules ont une faible longueur et que les tubes non accélérateurs (becs 40b, 41b et 42b) ont une longueur qui est 2 à 3 fois supérieure à celle de la distance inter-becs H.

Pour les protons de vitesse moyenne (entre 0,13 et 0,283 de la vitesse de la lumière) de la deuxième structure accélératrice à onde progressive directe, la distance L entre les cloisons transversales 43, 44 et 45 (figure 3) peut être augmentée et il en est de même de la longueur des becs 43b, 44b et 45b directe. Les distances L et H sont également données par le tableau "A" ci-après. Ce tableau fait apparaître que les cellules de la deuxième structure 13 sont plus longues que celles de la première structure 12 et que les tubes non accélérateurs (becs 43b, 44b et 45b) ont une longueur nettement supérieure à celle de la distance inter-becs H. Ceci est lié à la propagation directe en mode harmonique qui est utilisé.

Pour les protons de grande vitesse (0,283 à 0,566 de la vitesse de la lumière), l'espace d'accélération correspondant à H doit être augmenté pour tenir compte de l'augmentation de vitesse et la longueur des tubes non accélérateurs 46b, 47b et 48b (figure 4) devient nettement plus petite que la distance inter-becs H (tableau A). Ceci est lié à l'utilisation de la propagation directe en mode fondamental.

**TABLEAU A**

| proton | | mode harmonique | | | mode fondamental | dist. inter-becs |
|---|---|---|---|---|---|---|
| | | inverse | directe | | inverse | de l'ordre de |
| | | π/2+2π | π/2-2π | 3π/4-2π | 3π/4 | 2π/3 |
| T | β | L(mm) | L(mm) | L(mm) | L(mm) | H(mm) |
| 1 | 0,046 | 5,75 | | | | 1,53 |
| 2 | 0,065 | 8,12 | | | | 2,17 |
| 5 | 0,103 | 12,87 | | | | 3,43 |
| 10 | 0,145 | 18,12 | 10,87 | | | 4,83 |
| 20 | 0,203 | | 15,22 | | | 6,77 |
| 40 | 0,283 | | 21,22 | 17,68 | | 9,43 |
| 100 | 0,428 | | 32,10 | 26,75 | 16,1 | ≤ 14,3 |
| 200 | 0,566 | | | 35,37 | 21,4 | ≤ 18,9 |
| >> | 1,00 | | | | 37,5 | ≤ 33,3 |

Sur ce tableau "A", les deux premières colonnes définissent le faisceau de protons, par son énergie T et le rapport β = v/c entre sa vitesse v et la vitesse de la lumière C.

La troisième colonne définit la distance L pour une onde progressive inverse correspondant à un déphasage dit harmonique de (π/2+2π) tel que mis en oeuvre dans la première structure accélératrice 12.

La quatrième colonne définit la distance L pour une onde progressive directe en mode harmonique correspondant à un déphasage dit harmonique de (π/2-2π) tel que mis en oeuvre dans la deuxième structure accélératrice 13.

La cinquième colonne définit la distance L pour une onde progressive directe en mode harmonique correspondant à un déphasage dit harmonique de (3π/4-2π) qui peut être mis en oeuvre dans la troisième structure accélératrice 14, en remplacement de la propagation inverse en mode fondamental, dans le cas où la vitesse atteinte par les protons est comprise entre 0,283 et 0,566 de la vitesse de la lumière. Par ailleurs, la figure 12 montre schématiquement un accélérateur à protons utilisant une telle onde progressive directe en mode harmonique dans une structure accélératrice suivie d'une structure accélératrice à propagation inverse en mode fondamental correspondant à la structure 14 de la figure 1.

La sixième colonne définit la distance L pour une onde progressive inverse correspondant à un déphasage de 3π/4 qui est mis en oeuvre de préférence lorsque la vitesse des protons est supérieure à 0,423 de la vitesse de la lumière.

La septième colonne définit, de manière approximative, la distance inter-becs H pour les différentes valeurs de T et β. Cette distance sera définie plus précisément après un travail d'optimisation entre l'impédance shunt et le champ crête sur les becs.

Il est à noter que d'un déphasage à l'autre, les valeurs de L présentent des recoupements pour bien montrer que selon la vitesse des protons, on peut préférer une structure accélératrice à la précédente ou à la suivante.

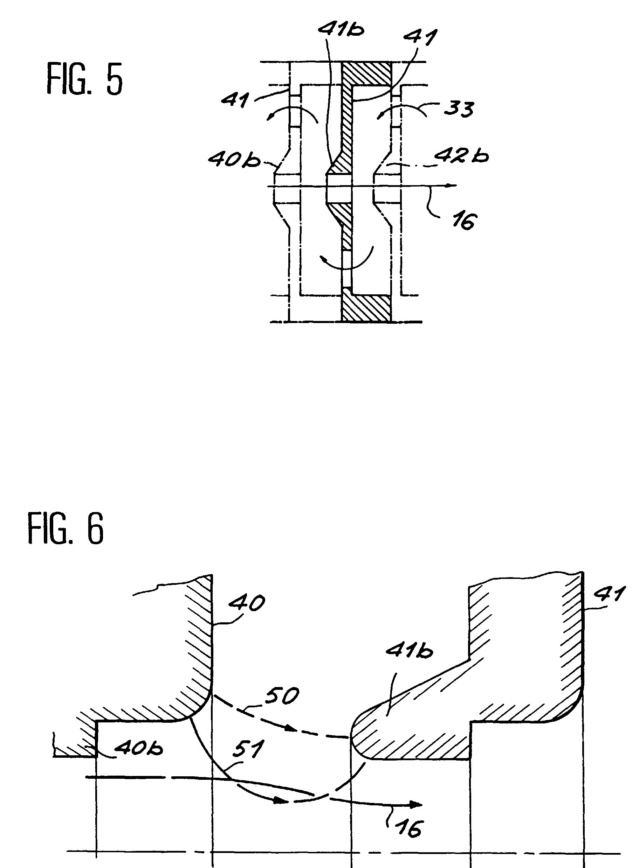

A la sortie de la source de protons 11, le faisceau de protons a tendance à diverger et à augmenter de diamètre; pour limiter l'augmentation de ce diamètre, l'invention propose d'effectuer une focalisation à l'intérieur de la première structure accélératrice 12 par une forme spéciale des becs 40b, 41b, et 42b de chaque cellule comme le montre les figures 5 et 6, cette dernière étant une coupe agrandie de deux becs successifs 40b et 41b. Dans la forme proposée de la figure 5, chaque bec est dissymétrique et n'existe en fait dans une cellule que du côté aval du faisceau 16, la partie côté amont ne comportant que la paroi transversale. Dans la forme proposée de la figure 6, la paroi transversale se termine par un retour dont le diamètre est plus grand que celui du bec. Cette dernière forme permet de créer un champ électrique E entre le retour inférieur de la paroi 40 et l'extrémité du bec 41b qui suit, dont les lignes de champ 50 et 51 focalisent la trajectoire (16) des protons vers l'axe.

Le tableau A montre que la longueur L des cellules de la première structure accélératrice 12 sont très petites (quelques millimètres) et donc difficiles à réaliser. L'invention propose alors d'introduire un déphasage supplémentaire de 2π et donc de porter le déphasage total de (π/2+2π) à (π/2+4π), ce qui correspond à un fonctionnement dit à l'harmonique supérieur 2, ce qui signifie que les protons restent dans les tubes non accélérateurs pendant deux périodes du signal hyperfréquence fourni par la source 22.

Il est également proposé d'introduire des glissements dans chaque cellule par l'utilisation de tubes disposés entre les parois de la cellule et dans lesquels passent le faisceau de protons 16. Dans le cas d'un faisceau d'énergie T = 1 MeV et β = 0,046, il est proposé d'utiliser quatre tubes 50, 51, 52 et 53 pour un glissement de 8π et un demi-tube 54 et 55 solidaire de chaque paroi pour obtenir un glissement supplémentaire de 2π. On obtient alors les structures des figures 8-a et 8-b dans lesquels chaque tube de glissement interne à la cavité est par exemple porté par un bras radial 50′ pour le tube 50, 51′ pour le tube 50 et 52′ pour le tube 52 (figure 8-b), chaque bras pouvant avoir une position angulaire différente autour de l'axe du faisceau 16.

Les tubes de glissements et leurs bras de support peuvent être remplacés par des tiges transversales 56 (figures 9-a et 9-b) percées de trous 57 suivant l'axe du faisceau 16.

Les tubes de glissement peuvent aussi être réalisés selon les schémas des figures 10-a , 10-b et 11-a, 11-b. Sur la figure 10-a, qui est une coupe longitudinale de deux tubes de glissement 58 et 58′, comportant chacun respectivement un rebord périphérique extérieur 59 et 59′, sont emmanchés en force dans un manchon diélétrique 60 qui est disposé entre les parois 61 et 62 de chaque cellule et maintenu en position concentrique sur lesdites parois par des encoches 69′. La figure 10-b est une coupe transversale selon l'axe BB′ de la figure 10-a. Sur la figure 11-a, deux tubes de glissement 63 et 63′ comportant chacun un rebord périphérique extérieur 64 et 64′ avec un épaulement 65 et 65′, sont maintenus en place par trois manchons diélectriques 66, 67 et 68, les manchons externes 66 et 68 étant maintenus en position concentrique sur les parois 61 et 62 de chaque cellule, par exemple par des encoches telles que celle référencée 69. La figure 11-b est une coupe transversale selon l'axe BB′ de la figure 11-a.

La figure 12 est une vue schématique d'un deuxième mode de réalisation d'un accélérateur de protons selon l'invention, analogue au premier mode dans la mesure où il comprend les trois structures accélératrices 12, 13 et 14, mais différent en ce qu'il comprend en outre une quatrième structure accélératrice 70 qui est disposée entre la structure 13 et la structure 14 et qui fonctionne en onde progressive directe en mode harmonique avec un déphasage (3π/4-2π).

Les caractéristiques L et H des cellules de la structure 70 sont données par la cinquième colonne du tableau A ci-dessus.

La structure 70 est alimentée par le klystron 22 par l'intermédiaire d'un guide d'onde 71 qui est la prolongation du guide d'onde 26 disposé à la sortie du coupleur 3 décibels référencé 27. Le guide d'onde 71 est connecté à la première cellule 73 de la structure 70 par un couplage magnétique réalisé à la périphérie de ladite cellule.

La dernière cellule 77 de la structure accélératrice 70 est connectée à la dernière cellule 76 de la structure accélératrice 14 par un guide d'onde 72 de manière à réaliser un couplage magnétique à la périphérie de ladite cellule 76.

Cette alimentation en série présente divers avantages qui sont décrits dans le brevet français 2 656 192 déposé le 14 décembre 1989 par la demanderesse.

Le faisceau de protons 16₂, issu de la sortie 19 de la structure 13 pénètre dans la structure 70 par un orifice d'entrée 74 et en sort (flèche 16′₃) par un orifice de sortie 75 pour pénétrer ensuite dans la structure 14 d'où il en sort (flèche 16′₄).

Dans l'exemple de réalisation de la figure 1 comportant trois structures accélératrices, la longueur totale le long de laquelle s'effectue l'accélération des protons est voisine de 12 mètres en utilisant un klystron de 70 mégawatts de puissance de crête.

Si le débit de la dose de protons que l'on doit fournir n'exige pas un courant accéléré trop important, on peut utiliser alternativement un système de compression des impulsions fournies par la source 22 pour transformer, par exemple, les impulsions de 4,5 microsecondes et de 45 mégawatts en des impulsions d'une microseconde de 140 mégawatts moyens environ; dans ce cas, la longueur totale pourra alors être réduite à six mètres.

## Revendications

1. Accélérateur linéaire de protons du type à onde progressive pour obtenir un faisceau de protons (16) d'une énergie déterminée qui comporte une source (11) fournissant un faisceau de protons (16), se propageant à une vitesse inférieure à la lumière, au moins une structure accélératrice du type à onde progressive à couplage magnétique (12, 13,14 ou 70) disposée de manière que le faisceau de protons issu de ladite source (11) pénètre dans ladite structure accélératrice (12, 13, 14, ou 70) et en sorte à l'énergie déterminée, et au moins un klystron (22) fournissant l'énergie haute fréquence à ladite structure accélératrice caractérisé en ce que :
- ladite structure accélératrice est constituée de cellules dont la longueur (L) est variable suivant la direction de propagation dudit faisceau de protons (16) afin de tenir compte de la variation de la vitesse (v) des protons au fur et à mesure de leur accélération dans ladite structure, et
- en ce que l'onde progressive à couplage magnétique est à propagation directe ou inverse, en mode fondamental ou harmonique.

2. Accélérateur selon la revendication 1, caractérisé en ce que le déphasage de l'onde progressive inverse en mode fondamental est de 3π/4.

3. Accélérateur selon la revendication 1, caractérisé en ce que le déphasage de l'onde progressive inverse en mode harmonique est de (π/2 + 2kπ), k étant un nombre entier égal ou supérieur à 1, le déphasage 2kπ étant obtenu par un allongement de l'espace de glissement entre les cellules adjacentes de ladite structure accélératrice.

4. Accélérateur selon la revendication 2 ou 3, caractérisé en ce que chaque cellule de ladite structure accélératrice comporte au moins un espace de glissement (50, 51, 52, 53, 58, 58′, 63, 63′) disposé entre les parois transversales (40, 41, 61, 62) de ladite cellule de manière à augmenter la longueur de cette dernière.

5. Accélérateur selon la revendication 4, caractérisé en ce que ledit espace de glissement (50, 51, 52, 53, 58, 58′, 63, 63′) entre les parois transversales de ladite cellule est réalisé par un tube métallique centré sur l'axe de propagation dudit faisceau de protons.

6. Accélérateur selon la revendication 5, caractérisé en ce que ledit tube métallique (50, 51, 52, 53) est porté par un bras (50′, 51′, 52′) solidaire de la paroi longitudinale de ladite cellule.

7. Accélérateur selon la revendication 5, caractérisé en ce que ledit tube métallique (58, 58′, 63, 63′) est porté par au moins une barre longitudinale (60) en matériau diélectrique qui prend appui sur les parois transversales (61, 62) de ladite cellule.

8. Accélérateur selon la revendication 5, caractérisé en ce que ledit tube métallique (58, 58′, 63, 63′) est porté par au moins un manchon (66, 67, 68) en matériau diélectrique qui prend appui sur les parois transversales (61, 62) de ladite cellule.

9. Accélérateur selon l'une des revendications 1 à 8, caractérisé en ce que le déphasage de l'onde progressive directe en mode harmonique est de (π/2 - 2kπ), k étant un nombre entier égal ou supérieur à 1, le déphasage 2kπ étant obtenu par un allongement de l'espace de glissement entre les cellules adjacentes de ladite structure accélératrice.

10. Accélérateur selon l'une des revendications 1 à 8, caractérisé en ce que le déphasage de l'onde progressive directe en mode harmonique est de (3π/4 - 2kπ), k étant un nombre entier égal ou supérieur à 1, le déphasage 2kπ étant obtenu par un allongement de l'espace de glissement entre les cellules adjacentes de ladite structure accélératrice.

11. Accélérateur selon la revendication 1, caractérisé :
- en ce qu'il comprend une première (12) et une deuxième (13) structures accélératrices disposées en série par rapport audit faisceau de protons (16),
- en ce que ladite première structure accélératrice est à onde progressive à propagation inverse en mode harmonique,
- en ce que ladite deuxième structure accélératrice est à onde progressive à propagation directe en mode harmonique,

12. Accélérateur selon la revendication 11, caractérisé
- en ce que lesdites première (12) et deuxième (13) structures accélératrices sont excitées par un klystron unique (22) connecté (24, 27, 25), d'une part, à la dernière cellule de ladite première structure accélératrice (12) et, d'autre part, à la première cellule de ladite deuxième structure accélératrice (13).

13. Accélérateur selon la revendication 12, caractérisé en ce que le klystron unique est connecté auxdites première (12) et deuxième (13) structures accélératrices par une cellule de couplage unique (29) disposée entre la dernière cellule de ladite première structure accélératrice (12) et la première cellule de ladite deuxième structure accélératrice (13).

14. Accélérateur selon l'une des revendications 11, 12 ou 13, caractérisé
- en ce qu'il comprend en outre une troisième structure accélératrice (70) disposée en série à la suite desdites première et deuxième structures accélératrices,
- en ce que ladite troisième structure accélératrice (70) est à onde progressive à propagation directe en mode harmonique.

15. Accélérateur selon l'une des revendications 11, 12 ou 13, caractérisé
- en ce qu'il comprend, en outre, une troisième structure accélératrice (14) disposée en série à la suite desdites première et deuxième structures accélératrices, et
- en ce que ladite troisième structure accélératrice (14) est à onde progressive à propagation inverse en mode fondamental.

16. Accélérateur selon la revendication 14, caractérisé
- en ce qu'il comprend, en outre, une quatrième structure accélératrice (14) disposée en série à la suite desdites première, deuxième et troisième structure accélératrices, et
- en ce que ladite quatrième structure accélératrice (14) est à onde progressive à propagation inverse en mode fondamental.

17. Accélérateur selon l'une des revendications 11 à 16, caractérisé en ce que lesdites structures accélératrices (12, 13, 70) sont alimentées en parallèle.

18. Accélérateur selon l'une des revendications 11 à 16, caractérisé en ce qu'au moins une (14) desdites structures accélératrices est alimentée en série.

19. Accélérateur selon l'une des revendications 11 à 16, caractérisé en ce que lesdites structures accélératrices (12, 13, 14, 70) sont alimentées en parallèle pour les unes (12, 13, 70) et en série pour les autres (14).

20. Accélérateur selon l'une des revendications 11 à 19 caractérisé en ce que lesdites structures accélératrices sont excitées (24, 25, 26, 72) par un klystron unique (22).

## Claims

1. Linear proton accelerator of the progressive wave type for producing a proton beam (16) of a given energy level, comprising a source (11) supplying a proton beam (16) propagated at a velocity below that of light, at least one accelerator structure of the progressive wave type having a magnetic coupler (12, 13, 14 or 70) arranged such that the proton beam from the said source (11) penetrates the said accelerator structure (12, 13, 14 or 70) and leaves it at a given energy level and at least one klystron (22) supplying high energy frequency at the said accelerator structure, characterised in that:
- the said accelerator structure consists of cells, the length (L) of which can be varied in the direction of propagation of the said proton beam (16) so as to take account of the variation in velocity (v) of the protons as they are accelerated in the said structure,
- the magnetically coupled progressive wave is propagated directly or inversely in fundamental or harmonic mode.

2. Accelerator as claimed in claim 1, characterised in that the phase shift of the inverse progressive wave is 3π/4 in fundamental mode.

3. Accelerator as claimed in claim 1, characterised in that the phase shift of the inverse progressive wave is (π/2 + 2kπ) in harmonic mode, where k is a whole number equal to or greater than 1, the phase shift 2kπ being produced by extending the drift space between the adjacent cells of the said accelerator structure.

4. Accelerator as claimed in claim 2 or 3, characterised in that each cell of the said accelerator structure has at least one drift space (50, 51, 52, 53, 58, 58', 63, 63') arranged between the transverse walls (40, 41, 61, 62) of the said cell so as to increase the length thereof.

5. Accelerator as claimed in claim 4, characterised in that the said drift space (50, 51, 52, 53, 58, 58', 63, 63') between the transverse walls of the said cell is provided by a metal tube centred on the axis of propagation of the said proton beam.

6. Accelerator as claimed in claim 5, characterised in that the said metal tube (50, 51, 52, 53) is mounted on an arm (50', 51', 52') integral with the longitudinal wall of the cell.

7. Accelerator as claimed in claim 5, characterised in that the said metal tube (58, 58', 63, 63') is supported by at least one longitudinal bar (60) of a dielectric material which is supported on the transverse walls (61, 62) of the said cell.

8. Accelerator as claimed in claim 5, characterised in that the said metal tube (58, 58', 63, 63') is supported by at least one sleeve (66, 67, 68) of a dielectric material which is supported on the transverse walls (61, 62) of the said cell.

9. Accelerator as claimed in one of claims 1 to 8, characterised in that the phase shift of the direct progressive wave is (π/2 - 2kπ) in harmonic mode, where k is a whole number equal to or greater than 1, the phase shift 2kπ being produced by extending the drift space between the adjacent cells of the said accelerator structure.

10. Accelerator as claimed in one of claims 1 to 8, characterised in that the phase shift of the direct progressive wave is (3π/4 - 21kπ) in harmonic mode, where k is a whole number equal to or greater than 1, the phase shift 2kπ being produced by extending the drift space between the adjacent cells of the said accelerator structure.

11. Accelerator as claimed in claim 1, characterised:
- in that it has first (12) and second (13) accelerator structures arranged in series relative to the said proton beam (16),
- in that the said first accelerator structure propagates a progressive wave inversely in harmonic mode,
- in that the said second accelerator structure propagates a progressive wave directly in harmonic mode.

12. Accelerator as claimed in claim 1, characterised
- in that the said first (12) and second (13) accelerator structures are excited by a single klystron (22) connected (24, 27, 25) to the last cell of the said first accelerator structure (12) on the one hand and to the first cell of the said second accelerator structure (13) on the other.

13. Accelerator as claimed in claim 12, characterised in that the single klystron is connected to the said first (12) and second (13) accelerator structures by a single coupler cell (29) arranged between the last cell of the said first accelerator structure (12) and the first cell of the said second accelerator structure (13).

14. Accelerator as claimed in one of claims 11, 12 or 13, characterised
- in that it has in addition a third accelerator structure (70) arranged in series after the said first and second accelerator structures,
- in that the said third accelerator structure (70) propagates a progressive wave directly in harmonic mode.

15. Accelerator as claimed in one of claims 11, 12 or 13, characterised
- in that it has in addition a third accelerator structure (14) arranged in series after the said first and second accelerator structures, and
- in that the third accelerator structure (14) propagates a progressive wave inversely in fundamental mode.

16. Accelerator as claimed in claim 14, characterised
- in that it has in addition a fourth accelerator structure (14), arranged in series after the said first, second and third accelerator structures, and
- in that the fourth accelerator structure (14) propagates a progressive wave inversely in fundamental mode.

17. Accelerator as claimed in one of claims 11 to 16, characterised in that at least one (14) of the said accelerator structures is shunt-fed.

18. Accelerator as claimed in one of claims 11 to 16, characterized in that at least one (14) of the said accelerator structures is series-fed.

19. Accelerator as claimed in one of claims 11 to 16, characterised in that some (12, 13, 70) of the said accelerator structures (12, 13, 14, 70) are shunt-fed and others (14) are series-fed.

20. Accelerator as claimed in one of claims 11 to 19, characterised in that the said accelerator structures are excited (24, 25, 26, 72) by a single klystron (22).

## Patentansprüche

1. Protonen-Linearbeschleuniger der mit Wanderwellen arbeitenden Bauart zum Erzielen eines Protonenstrahls (16) mit einer bestimmten Energie, umfassend eine Quelle (11), die einen Protonenstrahl (16) liefert, der sich mit einer Geschwindigkeit kleiner als die des Lichts fortpflanzt, zumindest eine Beschleunigerstruktur (12, 13, 14 oder 70) nach der mit Wanderwellen mit magnetischer Kopplung arbeitenden Bauart, die dergestalt angeordnet ist, daß der von der Quelle (11) abgegebene Protonenstrahl in die Beschleunigerstruktur (12, 13, 14 oder 70) eindringt und diese mit der bestimmten Energie verläßt, und zumindest ein Klystron (22), welches die Hochfrequenzenergie für die Beschleunigerstruktur liefert, dadurch gekennzeichnet, daß:
- die Beschleunigerstruktur aus Zellen besteht, deren Länge (L) entlang der Fortpflanzungsrichtung des Protonenstrahls (16) variabel ist, um der Schwankung der Geschwindigkeit (v) der Protonen je nach ihrer Beschleunigung in der Struktur Rechnung zu tragen, und
- daß sich die Wanderwelle mit magnetischer Kopplung als Grundschwingung oder harmonische Schwingung im Mit feld oder im Gegenfeld fortpflanzt.

2. Beschleuniger nach Anspruch 1, dadurch gekennzeichnet, daß die Phasenverschiebung der Gegenfeld-Wanderwelle mit der Grundschwingung etwa 3π/4 beträgt.

3. Beschleuniger nach Anspruch 1, dadurch gekennzeichnet, daß die Phasenverschiebung der harmonischen Gegenfeld-Wanderwelle (π/2 + 2kπ) beträgt, worin k eine Ganzzahl gleich oder größer als 1 ist und die Phasenverschiebung 2kπ durch eine Verlängerung des Triftraums zwischen den nebeneinanderliegenden Zellen der Beschleunigerstruktur erreicht wird.

4. Beschleuniger nach Anspruch 2 oder 3, dadurch gekennzeichnet, daß jede Zelle der Beschleunigerstruktur zumindest einen Triftraum (50, 51, 52, 53, 58, 58', 63, 63') umfaßt, der zwischen den Querwänden (40, 41, 61, 62) der Zelle angeordnet ist, um die Länge der letztgenannten zu vergrößern.

5. Beschleuniger nach Anspruch 4, dadurch gekennzeichnet, daß der Gleitraum (50, 51, 52, 53, 58, 58', 63, 63') zwischen den Querwänden der Zelle aus einem Metallrohr besteht, welches auf der Fortpflanzungsachse des Protonenstrahls zentriert ist.

6. Beschleuniger nach Anspruch 5, dadurch gekennzeichnet, daß das Metallrohr (50, 51, 52, 53) von einem mit der Längswand der Zelle verbundenen Arm (50', 51', 52') gehalten wird.

7. Beschleuniger nach Anspruch 5, dadurch gekennzeichnet, daß das Metallrohr (58, 58', 63, 63') durch zumindest einen Längsstab (60) aus dielektrischem Material getragen wird, der sich gegen die Querwände (61, 62) der Zelle abstützt.

8. Beschleuniger nach Anspruch 5, dadurch gekennzeichnet, daß das Metallrohr (58, 58', 63, 63') durch zumindest eine Muffe (66, 67, 68) aus dielektrischem Material getragen wird, die sich gegen die Querwände (61, 62) der Zelle abstützt.

9. Beschleuniger nach einem der Ansprüche 1 bis 8, dadurch gekennzeichnet, daß die Phasenverschiebung der harmonischen Mitfeld-Wanderwelle etwa (π/2 - 2kπ) beträgt, worin k eine Ganzzahl gleich oder größer als 1 ist und wobei die Phasenverschiebung 2kπ durch eine Verlängerung des Triftraums zwischen den nebeneinanderliegenden Zellen der Beschleunigerstruktur erzielt wird.

10. Beschleuniger nach einem der Ansprüche 1 bis 8, dadurch gekennzeichnet, daß die Phasenverschiebung der harmonischen Mitfeld-Wanderwelle etwa (3π/4 - 2kπ) beträgt, worin k eine Ganzzahl gleich oder größer als 1 ist und wobei die Phasenverschiebung 2kπ durch eine Verlängerung des Triftraums zwischen den nebeneinanderliegenden Zellen der Beschleunigerstruktur erzielt wird.

11. Beschleuniger nach Anspruch 1, dadurch gekennzeichnet,
- daß er eine erste (12) und eine zweite (13) Beschleunigerstruktur umfaßt, die bezüglich des Protonenstrahls (16) in Reihe angeordnet sind,
- daß die erste Beschleunigerstruktur mit einer harmonischen Gegenfeld-Wanderwelle arbeitet, und
- daß die zweite Beschleunigerstruktur mit einer harmonischen Mitfeld-Wanderwelle arbeitet.

12. Beschleuniger nach Anspruch 11, dadurch gekennzeichnet,
- daß die erste (12) und die zweite (13) Beschleunigerstruktur durch ein einziges Klystron (22) erregt werden, welches einerseits mit der letzten Zelle der ersten Beschleunigerstruktur (12) und andererseits mit der ersten Zelle der zweiten Beschleunigerstruktur (13) verbunden ist (24, 27, 25).

13. Beschleuniger nach Anspruch 12, dadurch gekennzeichnet, daß das einzige Klystron mit der ersten (12) und der zweiten (13) Beschleunigerstruktur durch eine einzige Koppelzelle (29) verbunden ist, die zwischen der letzten Zelle der ersten Beschleunigerstruktur (12) und der ersten Zelle der zweiten Beschleunigerstruktur (13) angeordnet ist.

14. Beschleuniger nach einem der Ansprüche 11, 12 oder 13, dadurch gekennzeichnet,
- daß er außerdem eine dritte Beschleunigerstruktur (70) umfaßt, die in Reihe auf die erste und die zweite Beschleunigerstruktur folgend angeordnet ist, und
- daß die dritte Beschleunigerstruktur (70) mit harmonischen Mitfeld-Wanderwellen arbeitet.

15. Beschleuniger nach einem der Ansprüche 11, 12 oder 13, dadurch gekennzeichnet,
- daß er außerdem eine dritte Beschleunigerstruktur (14) umfaßt, die in Reihe auf die erste und die zweite Beschleunigerstruktur folgend angeordnet ist, und
- daß die dritte Beschleunigerstruktur (14) mit Gegenfeld-Wanderwellen der Grundschwingung arbeitet.

16. Beschleuniger nach Anspruch 14, dadurch gekennzeichnet,
- daß er außerdem eine vierte Beschleunigerstruktur (14) umfaßt, die in Reihe auf die erste, die zweite und die dritte Beschleunigerstruktur folgend angeordnet ist, und
- daß die vierte Beschleunigerstruktur (14) mit Gegenfeld-Wanderwellen der Grundschwingung arbeitet.

17. Beschleuniger nach einem der Ansprüche 11 bis 16, dadurch gekennzeichnet, daß die Beschleunigerstrukturen (12, 13, 70) parallel versorgt werden.

18. Beschleuniger nach einem der Ansprüche 11 bis 16, dadurch gekennzeichnet, daß zumindest eine (14) der Beschleunigerstrukturen seriell versorgt wird.

19. Beschleuniger nach einem der Ansprüche 11 bis 16, dadurch gekennzeichnet, daß die Beschleunigerstrukturen (12, 13, 14, 70) zum Teil parallel (12, 13, 70) und zum Teil (14) seriell versorgt werden.

20. Beschleuniger nach einem der Ansprüche 11 bis 19, dadurch gekennzeichnet, daß die Beschleunigerstrukturen durch ein einziges Klystron (22) angeregt werden (24, 25, 26, 72).
